# EUROPEAN PATENT APPLICATION

(11) **EP 0 962 201 A1**
(43) Date of publication of application: **08.12.1999**
(21) Application number: 99500072.6
(22) Date of filing: 06.05.1999
(51) Int. Cl.: A61F 6/08

(54) **Unit for application of dose of chemical products and similar products**

(30) Priority: 03.06.1998 ES 9801150
(71) Applicant: Martinez Moreno, Julio Fernando, 28008 Madrid (ES)
(72) Inventor: Martinez Moreno, Julio Fernando, 28008 Madrid (ES)
(74) Representative: Carpintero Lopez, Francisco

(57) **Abstract**

It is composed of a body of cotton (1) in which a capsule (2) is established containing the product (3) in question, this capsule can be ruptured by simple pressure or can be equipped with an opening to empty its content, closed by means of a detachable lamina (4) to which a thread (5) is attached that, passing through the mass of cotton (1), emerges outside, in such a way that pulling said thread allows the lamina (4) to be removed to open the capsule in the moment in which it is used. A lint (6) acts as an envelope for the body of cotton (1) and from this a thread is attached (7) that facilitates the withdrawal of the unit from inside the vagina without introducing the fingers.

It is adapted to contain chemical products of any type including those that prevent infection by sexually transmitted diseases such as the AIDS virus HIV, syphilis, gonorrhoea, fungi, etc., and is equally suitable as a product that prevents pregnancy by destroying spermatozoids.

## Description

### OBJECT OF THE INVENTION

The present invention relates to a unit for application, preferably vaginal application, that has been specially conceived for certain chemical products, which prevent all types of infection by sexually transmitted diseases, such as the AIDS virus HIV, syphilis, gonorrhoea, fungi, etc., due to the disinfecting power of the contents of the chemical product, such as benzalcone chloride or similar compounds.

The object of the invention is to achieve a unit mono-dose that considerably facilitates the application of the product in question.

### BACKGROUND OF THE INVENTION

It is known in the state of the art the use of vaginal sponges that are impregnated with a spermicidal product that destroys spermatozoids, though these products are not always completely satisfactory in their function, and on the other hand they women find them difficult to use when introducing the product into and especially when removing the product from the vagina. This is accentuated by the fact that when the woman has badly cut nails or has scratches or cuts in her hands, the problems of introduction or removal to or from such a sensitive body part are made worse.

### DESCRIPTION OF THE INVENTION

The application unit proposed by the invention, on the basis of the previously described structure, has been conceived and structured in order to resolve the problem described above, allowing the easy opening of an interior blister or capsule, with minimum force, practically effortlessly, with perfectly controlled release of product. Furthermore, said application unit is designed for easy introduction into the vagina and an even easier removal, for which purpose it has a string bound to the outer part of the enveloping lint, which is pulled to withdraw the cotton without the need to introduce the fingers into the vagina.

For this and more particularly this application unit is structured as a capsule or blister that can be easily ruptured, with slight pressure, or incorporating an opening, that can even be introduced in the orifice of the filling of the capsule or blister, that is sealed with a small detachable lamina conveniently joined to a thread that, passing through the enveloping mass of cotton, emerges to the exterior, in such a way that when the application unit is used the aforementioned thread can be pulled so that the sealing lamina comes away and the product can be released to the outside soaking the cotton.

The aforementioned thread will preferably be of silk, with a view to avoiding the proliferation of bacteria therein, but could equally well be of another material without this affecting in any way the essence of the invention.

In accordance with another of the characteristics of the invention, the body or mass of cotton will be helped by an enveloping lint acting as configuring medium and container of said body, lint whose reticulated nature allows the product in question to be released, which was initially contained in the capsule or blister. To this outer lint a string of a certain length is joined that will be used to allow removal of the application unit from the vagina by pulling without the need to introduce the fingers into the vagina.

The chemical product contained in the blister or capsule will have high disinfecting power and will be appropriate for avoiding any sort of infection by sexually transmitted diseases such as the AIDS virus HIV, syphilis, gonorrhoea, fungi, etc, and will also be suitable for preventing pregnancies by destroying the spermatozoids, its most specific or ideal application being as vaginal cotton.

The chemical product contained in the capsule or blister is a product that has already shown its power for destroying viruses or bacteria, such as benzalcone chloride or a mixture thereof with other suitable products.

### DESCRIPTION OF THE DRAWINGS

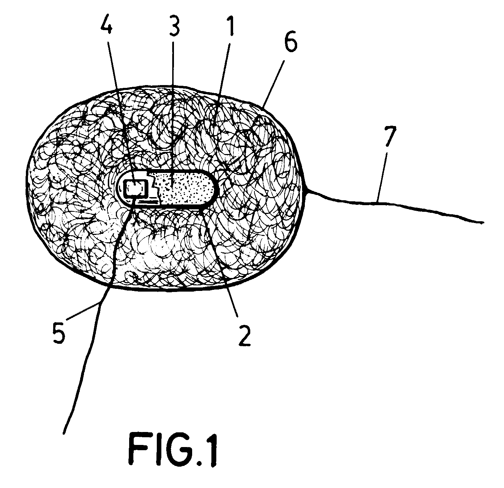

To complement the description that is being made and with a view to facilitate a better understanding of the characteristics of the invention, in accordance with an example of practical realisation thereof, as an integral part of said description, a single sheet is included of illustrative and non-limiting character, and in its only figure, a schematic representation has been made of a section of an application unit of a unit dose of health products and similar products carried out in accordance with the object of the present invention.

### PREFERRED EMBODIMENT OF THE INVENTION

From this figure it can be observed how the application unit proposed by the invention is constituted of a body or mass of cotton (1), that can adopt a spherical configuration but that will preferably adopt a flattened form, containing within it a blister or capsule (2) containing the chemical product (3) in question, a product that, as was discussed earlier, will preferably be a disinfectant capable of eliminating both viruses and bacteria of any type, as well as also being a product that can prevent pregnancy by destroying the spermatozoids. This product will preferably be a chemical product with high efficiency such as benzalcone chloride or a mixture thereof with other products.

From this basic structure, the application unit of the invention incorporates, in any suitable point of its capsule or blister (2), an exit orifice for the product (3) contained inside, an orifice that will normally be sealed by a small detachable lamina (4), attached to the capsule (2) by any appropriate means, for example by adhesive, said detachable lamina extending into a thread (5) with a suitable length so that its free end emerges outside passing completely through the cotton body (1). Furthermore, a length emerges that can be easily held to allow pulling to release the detachable lamina (4).

In this way the application unit maintains its capsule (2) preferably sealed until the moment in which it is used, the moment in which the opening is very easy and quick, not needing more than a gentle force, with the need to apply manual pressure to the unit to achieve the "bursting" of the said capsule (2), and so the release of the product to the outside is perfectly controlled and the cotton body (1) becomes uniformly impregnated, without loss of product or its loss to the outside as a squirt of liquid.

As a complement to the described structure and as is observed in the figure, the body of cotton (1) is enveloped by an enveloping exterior (6) based on lint, that stabilises and protects it, as well as serving as an element to which a second immovable string (7) is attached which is used for withdrawing this application unit from the vagina as if it were a tampon, thus avoiding the introduction of fingers inside the vagina.

Even so, and as has been mentioned earlier, to said exit orifice, with its detachable sealing lamina (4) and with its pulling thread (5) can be non-existent, depending on the type of application considered for the unit, in which case the capsule (2) will be opened by "bursting" thereof, particularly by applying a certain degree of pressure thereto.

## Claims

1. Unit for application of dose of chemical products and similar products, such as disinfectants capable of eliminating all types of viruses and bacteria, disinfectants in general, and even spermicides, or other similar products, characterised in that it consists of a body or small body of cotton (1) in which a capsule or blister is established (2) containing the chemical and/or similar product (3) in question, a capsule capable of allowing the release of chemical product.

2. Unit for application of dose of chemical products or similar products, according to claim 1, characterised in that the body or mass of cotton (1) is bordered by a enveloping lint (6), to which a string is attached (7) that helps the withdrawal of this application unit from the interior of the vagina.

3. Unit for application of dose of chemical products and similar products, according to the previous claims, characterised in that the material of which the capsule or blister (2) is made is of breakable nature when pressure is applied.

4. Unit for application of a dose of chemical products and similar products, according to claims 1 and 2, characterised in that the capsule (2) containing the chemical product (3) incorporates an opening at any suitable point thereof, sealed with a small detachable lamina (4), this lamina having had a thread (5) attached to it, of a suitable length to pass through the cotton body (1) and emerge outside, consisting of a loop that allows it to be manually pulled to detach the lamina (4) in the moment when the unit is used.

5. Unit for application of a dose of chemical products and similar products, according to claim 4, characterised in that the thread (5) for pulling the detachable lamina (4), of any nature, will preferably be of silk.

6. Unit for application of dose of chemical products and similar products, according to the preceding claims, characterised in that the chemical product (3) contained in the capsule (2) is benzalcone chloride.
